## Europäisches Patentamt

## European Patent Office

## Office européen des brevets

(19)

(11) Veröffentlichungsnummer: **0 023 303**
**B1**

(12) **EUROPÄISCHE PATENTSCHRIFT**

(45) Veröffentlichungstag der Patentschrift:
**13.04.83**

(21) Anmeldenummer: **80104039.5**

(22) Anmeldetag: **12.07.80**

(51) Int. Cl.³: **C 07 C 1/24, C 07 C 11/02, B 01 J 29/38**

(54) Verfahren zur Herstellung niederer Olefine aus Methanol/Wasser-Gemischen.

(30) Priorität: **18.07.79 DE 2928922**

(43) Veröffentlichungstag der Anmeldung:
**04.02.81 Patentblatt 81/5**

(45) Bekanntmachung des Hinweises auf die Patenterteilung:
**13.04.83 Patentblatt 83/15**

(84) Benannte Vertragsstaaten:
**BE DE FR GB IT NL**

(73) Patentinhaber: **HOECHST AKTIENGESELLSCHAFT,
Postfach 80 03 20, D-6230 Frankfurt/Main 80 (DE)**

(72) Erfinder: **Wunder, Friedrich, Dr., Jahnstrasse 46,
D-6093 Flörsheim am Main (DE)**
Erfinder: **Leupold, Ernst Ingo, Dr., Am Zäunefeld 15,
D-6392 Neu-Anspach (DE)**
Erfinder: **Hachenberg, Horst, Dr., Mohnweg 1,
D-6229 Walluf (DE)**
Erfinder: **Schmidt, Hans-Joachim, Dr., Am Burgenblick 6,
D-6240 Königstein/Taunus (DE)**

(56) Entgegenhaltungen:
**DE-A-2 755 229
US-A-4 148 750
Chemical Abstracts Band 89, Nr. 12, 1978 Columbus, Ohio, USA. T. I. OSIPOVA et al., »Prediction of conditions for separation of cations on anoin exchangers in ethylenediaminetetraacetate form«, Seite 336, rechte Spalte, Abstract Nr. 95415p.
Chemical Abstracts Band 73, Nr. 16, 1970 Columbus, Ohio, USA. K. MAREK et al., »Acid two-component bath for recovery of cation exchangers«, Seite 267, linke und rechte Spalten, Abstract Nr. 81025u.**

**Chemical Abstracts, Band 81, Nr. 6, 1974, Columbus, Ohio, USA. Y. SAKURAI et al., »Detergents for ion exchange resins«, Seite 45, rechte Spalte, Abstract Nr. 26520s.
Chemical Abstracts Band 71, Nr. 16, 1969 Columbus, Ohio, USA. H. VAHEMETS et al., »Effect of complexing on the regeneration of a strongly acidic cation-exchange resin«, Seite 343, linke Spalte, Abstract Nr. 74475w.**

## Verfahren zur Herstellung niederer Olefine aus Methanol/Wasser-Gemischen

In der DE-OS 2 755 229 wird ein Verfahren zur Herstellung von niederen Olefinen aus Methanol und/oder Dimethylether beschrieben, bei dem die Umsetzung von Methanol an Mangan enthaltenden Aluminiumsilikat-Katalysatoren erfolgt. Diese Katalysatoren müssen periodisch regeneriert, d. h. von gebildeten Nebenprodukten befreit werden, was schon bei relativ niedrigen Temperaturen von 300 bis 500° C, am vorteilhaftesten bei der Reaktionstemperatur selbst, mit Luft oder anderen sauerstoffhaltigen Gasen erfolgen kann. Bei Einsatz von Methanol, das kein oder nur wenig Wasser enthält, sind diese Katalysatoren sehr oft regenerierbar, ohne daß eine Leistungs- oder Selektivitätsminderung auftritt. Jedoch fällt bei der beschriebenen Reaktion ein Wasser-Methanolgemisch an, wenn der Umsatz nicht vollständig ist. Das in diesem Gemisch vorliegende Methanol muß zurückgewonnen werden. Es fällt jedoch bei einer Destillation, falls man nicht erheblichen Aufwand treibt, nur ein mehr oder weniger wasserhaltiges Methanol an. Die Gegenwart von Wasser wirkt sich zwar günstig auf die Selektivität von Ethylen aus — besonders der Butenanteil wird verringert — jedoch zeigte sich, daß einige Mangan-Alumosilikat-Katalysatoren unter den Reaktionsbedingungen deutlich an Aktivität verlieren und nur wenige Male wieder regenerierbar sind. Eine sorgfältige Entwässerung des Methanols vor der Rückführung löst zwar dieses Problem, bedeutet jedoch einen hohen Energieaufwand.

Somit stellte sich die Aufgabe, einen unter Reaktionsbedingungen gegen größere Mengen Wasser stabilen Katalysator zu entwickeln.

Es wurde nun ein Verfahren zur Herstellung von $C_2$—$C_4$-Olefinen aus Methanol und/oder Dimethylether in Gegenwart von Wasser an einem Mangen enthaltenden Aluminiumsilikat Katalysator gefunden, dadurch gekennzeichnet, daß der Katalysator mit einer Lösung von Ethylendiamintetraessigsäure oder Weinsäure mit einem pH von 3 bis 7 ausgewaschen wird. Bevorzugt ist ein pH von 4 bis 5. Vorzugsweise wird das Auswaschen vor dem Aufbringen des Mangans vorgenommen. Besonders geeignet ist das Auswaschen mit Ethylendiamintetraessigsäure-Lösung.

Als Aluminiumsilikate kommen beispielsweise die üblichen, amorphen sauren Crackkatalysatoren in Frage, die im allgemeinen etwa 13 bis 25 Gew.-% Aluminiumoxid und 75 bis 87 Gew.-% Siliciumoxid enthalten. Zum weiteren sind auch natürliche oder synthetische kristalline Aluminiumsilikate geeignet, wie sie z. B. unter Bezeichnungen wie Faujasite, Zeolithe, Chabasite, Analcim, Gismondit, Gmelinit, Natrolith, Mordenite und Erionite oder auch allgemein als Molsiebe bekannt sind.

Bei den kristallinen Molsieben mit unterschiedlichen Porendurchmessern ist es zweckmäßig, solche mit großen Poren, z. B. Poren von 5 Å und höher zu verwenden.

Zur Herstellung der erfindungsgemäßen Katalysatoren werden die Aluminiumsilikate vor oder nach dem Aufbringen des Mangans mit einer Lösung von Ethylendiamintetraessigsäure oder Weinsäure, die mit einer Base auf pH 3—7, vorzugsweise auf pH 4—5, eingestellt wurde, gewaschen. Als Basen eignen sich z. B. Lithiumhydroxyd, Natriumhydroxyd, Kaliumhydroxyd, Rubidiumhydroxyd und Caesiumhydroxyd, besonders das Natriumhydroxyd und das Kaliumhydroxyd. Auch Alkali-Salze schwacher Säuren, wie die Carbonate eignen sich.

Die Konzentration der Lösungen von Ethylendiamintetraessigsäure oder Weinsäure können in weiten Grenzen variieren, von ca. 1%iger Lösung bis zu gesättigter Lösung; bevorzugt werden Lösungen, die bei Zimmertemperatur in etwa gesättigt sind. Die Temperatur dieser Lösungen liegt vorzugsweise zwischen 0° C und 50° C. Als Lösungsmittel sind bevorzugt Wasser, Methanol, Formamid, Dimethylformamid oder deren Gemische, insbesondere Wasser. Nach dem Auswaschen mit Ethylendiamintetraessigsäure- oder Weinsäure-Lösung wird der Katalysator zur Entfernung der Ethylendiamintetraessigsäure bzw. Weinsäure mit reinem Lösungsmittel gewaschen. Das Aktivieren des erfindungsgemäßen Katalysators erfolgt vorzugsweise durch anschließendes — ggf. aber auch vorhergehendes — Aufbringen von 0,1 bis 10 Gew.-% Mangen in Form von Mangansalzlösungen auf das Aluminiumsilikat. Dazu kann man beispielsweise das Aluminiumsilikat mit einer Lösung von Mangansalzen tränken und dann trocknen. Als Lösemittel für die Mangansalze sind Wasser, Methanol, Formamid, Dimethylformamid oder auch deren Gemisch bevorzugt, insbesondere Wasser. Auch durch längeres Einwirken einer Mangansalzlösung auf das Aluminiumsilikat und anschließendes Nachwaschen mit reinem Lösemittel und Trocknen kann Mangan aufgebracht werden.

Bei Verwendung von Molsieben kann eine der bei diesen Materialien üblichen Methoden der Imprägnierung mit einem Metallkation gewählt werden; das kann ein Austausch der ursprünglich auf dem Molsieb vorhandenen Kationen gegen Mangan sein, es kann auch eine vorgelagerte Überführung des Molsiebes in die Protonenform mit anschließender Behandlung mit der Lösung eines Mangansalzes sein.

Es hat sich weiterhin oft als vorteilhaft für eine hohe Selektivität erwiesen, zusätzlich zum Mangan noch weitere Elemente als Cokatalysatoren zu verwenden. Als solche sind Elemente geeignet, die in ihren Verbindungen ein-, zwei- oder dreiwertig vorkommen, wie beispielsweise die Alkalimetalle (insbesondere Lithium, Natrium und Kalium), die Erdalkalimetalle (insbesondere Magnesium und Calcium), Zink, Lanthan, Seltene Erden (wie Praseodym, Neodym, Samarium, Gadolinium oder auch ihre Mischungen, wie im Didymium) und Beryllium.

Die cokatalytisch wirksamen, weiteren Metallsalze können gleichzeitig mit dem Mangansalz aufgebracht werden, z. B. indem man eine Lösung von Mangansalz mit einer Lösung eines oder mehrerer der anderen Metallsalze mischt und dieses Gemisch einwirken läßt.

Sie können aber auch nacheinander auf das Aluminiumsilikat gebracht werden.

Als Mangansalze kommen alle löslichen Salze in Frage, z. B. Chlorid, Sulfat, Nitrat, Formiat, Acetat, Propionat, Butyrat, Lactat, Citrat, Tartrat und Salze der Apfelsäure. Entsprechendes gilt für die Cokatalysatoren. Verwendet man gemeinsame Lösungen von Mangan und cokatalytisch wirksamem Element, so ist die gegenseitige Löslichkeitsbeeinflussung zu berücksichtigen, d. h. bei Einsatz von Calcium oder Barium ist die Verwendung von Sulfat als Anion unzweckmäßig.

Die Katalysatoren werden nach der Imprägnierung bei Normaldruck, Vakuum oder Überdruck bei Normaltemperatur oder erhöhten Temperaturen getrocknet. Im allgemeinen liegen die Trocknungstemperaturen unterhalb von 600° C, vorzugsweise zwischen 100 und 200° C.

Bei Einsatz von Methanol als Ausgangsprodukt kann man entweder Methanol direkt über den erfindungsgemäßen Katalysator leiten oder aber es zunächst in einer vorgeschalteten Dehydratisierungsreaktion an einem üblichen Dehydratisierungskatalysator, wie Aluminiumoxid oder Aluminiumsilikat, in Dimethyläther überführen und diesen dann über den erfindungsgemäßen Katalysator leiten.

Man kann aber auch Gemische von Methanol und Dimethyläther oder Dimethyläther allein als Ausgangssubstanz verwenden.

Die Einsatzkomponenten Methanol und/oder Dimethyläther können auch mit Inertgasen verdünnt in die Reaktion eingesetzt werden. Zur Erniedrigung des Partialdruckes sind beispielsweise Stickstoff, Kohlendioxid, Alkene geeignet. Die Reaktion kann aber zu diesem Zweck auch bei vermindertem Druck bis herab zu 0,1 bar durchgeführt werden.

Der Wassergehalt der Einsatzprodukte kann in weiten Grenzen variieren, von wasserfrei bis zu etwa 80% Wasser, wobei jedoch höhere Wassermengen höhere Verdampfungs- und Destillationskosten hervorrufen.

Die Reaktionstemperatur liegt im allgemeinen zwischen 300 und 500° C, bevorzugt zwischen 350 und 450° C, besonders bevorzugt zwischen 380 und 420° C. Wählt man die Reaktionsbedingungen so, daß nur ein unvollständiger Umsatz an Methanol und/oder Dimethyläther erreicht wird, so können die unumgesetzten Anteile abgetrennt und zurückgeführt werden.

Die nach dem erfindungsgemäßen Verfahren hergestellten Alkene können nach üblichen Methoden, z. B. durch Destillation, von den als Nebenprodukt entstehenden Alkanen und voneinander getrennt werden.

Damit steht ein Verfahren zur Verfügung, das in besonders selektiver und damit wirtschaftlicher Weise die Herstellung von industriell bedeutenden niederen Alkenen aus Methanol und/oder Dimethylether in Gegenwart von Wasser gestattet. Der erfindungsgemäße Katalysator kann in überraschend einfacher Weise aus leicht zugänglichen Substanzen hergestellt werden.

Die folgenden Beispiele erläutern das erfindungsgemäße Verfahren:

### Vergleichsbeispiel 1

Es werden 300 ml eines handelsüblichen Chabasit-Erionit-Gemisches in Form von Strangpreßlingen mit 300 ml gesättigter, wäßriger Manganacetatlösung überschichtet, nach 48 h mit Wasser ausgewaschen und getrocknet. Man erhält 202 g Katalysator mit 3,6% Mn. Über diesen Katalysator werden stündlich 89,1 g Methanol bei 400° C geleitet. Man erhält 25,8 l Gas je Stunde bestehend aus

31,0 Gew.-% Ethylen
32,5 Gew.-% Propylen
5,4 Gew.-% Butene
6,8 Gew.-% Methan
1,4 Gew.-% Ethan
19,3 Gew.-% Propan
3,4 Gew.-% Butan
0,3 Gew.-% Sonstiges

sowie 4,5 g Dimethylether, 9,2 g Methanol und 43,3 g Wasser. Dies entspricht einem 89,6%igen Umsatz von Methanol, einer Selektivität zu $C_2-C_4$-Olefinen von 68,8% und einer Selektivität zu $C_2-C_4$-Kohlenwasserstoffen von 93%, wenn der gebildete Dimethylether und das nicht umgesetzte Methanol zurückgeführt werden.

Bei nachlassender Leistung wird der Katalysator durch Überleiten von Luft bei 430° C regeneriert, wobei die Leistung des frischen Katalysators erreicht wird. Auch nach 26 Regenerationscyclen wird keine Ermüdung des Katalysators beobachtet.

### Vergleichsbeispiel 2

Man wiederholt Vergleichsbeispiel 1 mit dem einzigen Unterschied, daß dem Einsatz-Methanol noch 45,4 g/h Wasser zugegeben werden. Man erhält bei einem Einsatz von stündlich 92,3 g Methanol und 45,4 g Wasser 26,2 l Gas je Stunde mit

34,2 Gew.-% Ethylen
33,7 Gew.-% Propylen
6,3 Gew.-% Butene
7,2 Gew.-% Methan
1,3 Gew.-% Ethan
14,7 Gew.-% Propan

2,4 Gew.-% Butan
0,2 Gew.-% Sonstiges

sowie 4,3 g Dimethylether, 8,9 g Methanol und 44,2 g Wasser je Stunde. Dies entspricht einem Methanol-Umsatz von 98,7%, einer Selektivität zu $C_2$—$C_4$-Olefinen von 74,2% und einer Selektivität zu $C_2$—$C_4$-Kohlenwasserstoffen von 92,6%, wenn Dimethylether und nicht umgesetztes Methanol zurückgeführt werden.

Bereits nach der — wie in Vergleichsbeispiel 1 durchgeführten — dritten Regeneration werden nur noch Kohlenwasserstoff-Selektivitäten von 12% erreicht, d. h., daß der Zusatz von Wasser den Katalysator irreversibel schädigt.

Beispiel

Man läßt 300 ml einer käuflichen Chabasit-Erionit-Mischung in Strangpreßlingen (das gleiche Molekularsieb wie in den beiden vorhergehenden Vergleichsbeispielen) 48 h in einer gesättigten Lösung von Ethylendiamintetraessigsäure-Di-Natriumsalz von pH 4,45 bei Zimmertemperatur (25° C) stehen, wäscht anschließend aus und tauscht wie in den vorhergehenden Beispielen mit Mangan aus. Unter den Bedingungen von Vergleichsbeispiel 2 erhält man bei einem Einsatz von stündlich 57,5 g Methanol und 57,5 g Wasser im Reaktionsprodukt 7,3 g unumgesetztes Methanol, 73,8 g Wasser, 5,8 g Dimethylether und 14 l eines Kohlenwasserstoffgemisches bestehend aus

42,2 Gew.-% Ethylen
37,6 Gew.-% Propylen
5,0 Gew.-% Butene
7,0 Gew.-% Methan
1,6 Gew.-% Ethan
4,9 Gew.-% Propan
1,2 Gew.-% Butane
0,5 Gew.-% Sonstiges

Dies entspricht einem Umsatz von 87,3%, einer Selektivität von 84,8% zu $C_2$—$C_4$-Olefinen und einer Selektivität von 92,5% zu $C_2$—$C_4$-Kohlenwasserstoffen, wenn nichtumgesetztes Methanol und gebildeter Dimethylether zurückgeführt werden.

Nach 38 wie in Vergleichsbeispiel 1 durchgeführten Regenerationscyclen ist kein Nachlassen der Leistung feststellbar, es werden die Leistungen des frischen Katalysators erreicht.

**Patentansprüche**

1. Verfahren zur Herstellung von $C_2$—$C_4$-Olefinen aus Methanol und/oder Dimethylether in Gegenwart von Wasser an einem Mangan enthaltenden Aluminiumsilikat-Katalysator, dadurch gekennzeichnet, daß der Katalysator mit einer Lösung von Ethylendiamintetraessigsäure oder Weinsäure mit einem pH von 3 bis 7 ausgewaschen wird.

2. Verfahren nach Anspruch 1, dadurch gekennzeichnet, daß die Lösung einen pH von 4—5 hat.

3. Verfahren nach Anspruch 1 oder 2, dadurch gekennzeichnet, daß das Auswaschen vor dem Aufbringen des Mangans auf das Aluminiumsilikat vorgenommen wird.

4. Verfahren nach einem der Ansprüche 1 bis 3, dadurch gekennzeichnet, daß das Auswaschen mit einer Lösung von Ethylendiamintetraessigsäure vorgenommen wird.

5. Verfahren nach einem der Ansprüche 1 bis 4, dadurch gekennzeichnet, daß der pH mit Alkalihydroxid eingestellt wird.

6. Verfahren nach einem der Ansprüche 1 bis 5, dadurch gekennzeichnet, daß der pH mit Natrium- oder Kaliumhydroxid eingestellt wird.

**Claims**

1. Process for the manufacture of $C_2$—$C_4$-olefins from methanol and/or dimethyl ether in the presence of water, on an aluminum silicate catalyst containing manganese, characterized by washing the catalyst with a solution of ethylenediaminetetraacetic acid or tartaric acid with a pH of 3 to 7.

2. Process as claimed in claim 1, wherein the solution has a pH of 4—5.

3. Process as claimed in claim 1 or 2, wherein the washing is carried out before the application of the manganese to the aluminum silicate.

4. Process as claimed in any of claims 1 to 3, wherein the washing is carried out with a solution of ethylenediaminetetraacetic acid.

5. Process as claimed in any of claim 1 to 4, wherein the pH is established with alkali metal hydroxide.

6. Process as claimed in any of claims 1 to 5, wherein the pH is established with sodium hydroxide or potassium hydroxide.

**Revendications**

1. Procédé de préparation d'oléfines en $C_2$—$C_4$ à partir de méthanol et/ou d'oxyde de diméthyle, en présence d'eau, au moyen d'un catalyseur à base d'un silicate d'aluminium contenant du manganèse, procédé caractérisé en ce qu'on lave le catalyseur avec une solution d'acide éthylène-diamine-tétacétique ou d'acide tartrique ayant un pH de 3 à 7.

2. Procédé selon la revendication 1, caractérisé en ce que la solution a un pH de 4 à 5.

3. Procédé selon l'une des revendications 1 et 2, caractérisé en ce que le lavage est effectué avant l'application du manganèse sur le silicate d'aluminium.

4. Procédé selon l'une quelconque des revendications 1 à 3, caractérisé en ce que le lavage est effectué avec une solution d'acide éthylène-diamine-tétracétique.

5. Procédé selon l'une quelconque des revendications 1 à 4, caractérisé en ce que le pH est ajusté au moyen d'un hydroxyde de métal alcalin.

6. Procédé selon l'une quelconque des revendications 1 à 5, caractérisé en ce que le pH est ajusté au moyen d'hydroxyde de sodium ou d'hydroxyde de potassium.